# EUROPEAN PATENT APPLICATION

(11) **EP 3 075 857 A1**
(43) Date of publication of application: **05.10.2016**
(21) Application number: 16163069.4
(22) Date of filing: 31.03.2016
(51) Int. Cl.: C12N 15/63, C07K 14/435, C07K 16/40, C12P 21/00

(54) **METHOD OF PRODUCING GLYCOPROTEIN, AND VECTOR, KIT, INSECT ORGANISM AND INSECT CELLS**

(30) Priority: 01.04.2015 JP 2015075070
(71) Applicant: SYSMEX CORPORATION, Kobe-shi, Hyogo 651-0073 (JP)
(72) Inventor: Higa, Yukiko, Kobe-shi, Hyogo, 651-0073 (JP); Kataoka, Yukiko, Kobe-shi, Hyogo, 651-0073 (JP); Nomura, Tsuyoshi, Kobe-shi, Hyogo, 651-0073 (JP); Suganuma, Masatoshi, Kobe-shi, Hyogo, 651-0073 (JP)
(74) Representative: Paemen, Liesbet R.J.

(57) **Abstract**

Disclosed is a method of producing a glycoprotein, the method including the steps of: introducing a gene encoding a desired protein and a gene encoding an antibody that inhibits a decomposing enzyme preventing formation of a desired complex-type sugar chain in the desired protein into an insect organism or insect cells; and obtaining a desired protein having a desired complex-type sugar chain from the insect organism or insect cells obtained in the introduction step.

## Description

### TECHNICAL FIELD

The present invention relates to a method of producing a glycoprotein. More specifically, the present invention relates to a method of producing a glycoprotein having a desired complex-type sugar chain and a vector, kit, and insect organism and insect cells used in the production method.

### BACKGROUND

In many cases, proteins expressed in organisms are in the form of glycoproteins having several to several tens of relatively short sugar chains added. Since sugar chains are added, many of these glycoproteins are known to confer specific properties thereon. In recent years, it has become clear that sugar chain components of glycoproteins have an important role in metastasis of cancer, immunization or the like. Attention has been concentrated on the study focused on the potential usefulness of the sugar chain components. For example, it is assumed that when an antibody drug having strong antibody-dependent cellular cytotoxicity alone can be screened using a glycoprotein having a sugar chain characteristic to cancer, dependence on chemotherapy is decreased, and thus it is possible to provide a new cancer therapy with few side effects.

In order to develop the study on sugar chains, it is essential to mass-produce glycoproteins having a desired sugar chain added. For the mass production of glycoproteins having a mammal-type sugar chain, the use of an insect system has been recently researched. However, the sugar chain added to the protein expressed in an insect is a mannose-core-type N-linked sugar chain in many cases. The mannose-core-type N-linked sugar chain has a structure in which two different mannoses are respectively β-1,3-linked and β-1,6-linked to 3-position and 6-position of the mannose β-1,4-linked to the diacetylchitobiose moiety at the reducing end. In the insect, a sugar chain with N-acetyl glucosamine linked to the non-reducing end of the mannose-core-type sugar chain may be formed in the sugar chain forming process. However, this sugar chain eventually loses N-acetyl glucosamine due to the effect of N-acetyl glucosaminidase and becomes a mannose-core-type sugar chain.

On the other hand, a sugar chain observed in mammals such as humans is mainly a complex-type N-linked sugar chain. Such a complex-type N-linked sugar chain includes a sugar chain in which N-acetyl glucosamine is linked to the non-reducing end of the mannose-core-type sugar chain, a sugar chain in which galactose is linked to the above sugar chain, and a sugar chain in which sialic acid is linked to the above sugar chain. Therefore, when a glycoprotein having a mammal-type sugar chain is produced in an insect system, it is necessary to introduce galactose transferase, sialyltransferase or the like into an insect. In order to further improve the production efficiency of the glycoprotein having a mammal-type sugar chain, the suppression or inhibition of activity of N-acetylglucosaminidase expressed in the insect is assumed to be effective.

US2004/203117 describes a method of inhibiting N-acetylglucosaminidase activity by 2-acetamide-1,2-dideoxynojirimycin (2-ADN) in order to add sialic acid to a sugar chain in insect cells. As other methods of inhibiting N-acetylglucosaminidase activity, Yudai NAGATA et al., Influence of Knock Down of "Bombyx mori β-N-acetylglucosaminidase (BmFDL) on N-glycan formation", Graduate School, Kyushu University, Molecular Biology Society of Japan, December 13, 2011 describes a method of inhibiting N-acetylglucosaminidase activity by knocking down Bombyx mori fused lobes (BmFDL), and Ayumi KANEMATSU et al., "Suppression of Expression of N-acetylglucosaminidase Gene by Expression of shRNA in Bombyx mori Cells and Larvae" Shizuoka University, Molecular Biology Society of Japan, September 25, 2012 describes a method of inhibiting N-acetylglucosaminidase activity by using shRNA.

However, an N-acetylglucosaminidase inhibitor (2-acetamide-1,2-dideoxynojirimycin; 2-ADN) used in US2004/203117 is expensive. When the N-acetylglucosaminidase inhibitor is particularly applied to a silkworm organism, it is supposed to be necessary to administer the inhibitor multiple times at constant intervals in order to maintain the effects because of excretion or decomposition of the inhibitor due to metabolism. In order to ensure the inhibition, it is necessary to optimize the culture conditions, which is cumbersome. The methods described in Yudai NAGATA et al., Influence of Knock Down of "Bombyx mori β-N-acetylglucosaminidase (BmFDL) on N-glycan formation", Graduate School, Kyushu University, Molecular Biology Society of Japan, December 13, 2011 and Ayumi KANEMATSU et al., "Suppression of Expression of N-acetylglucosaminidase Gene by Expression of shRNA in Bombyx mori Cells and Larvae" Shizuoka University, Molecular Biology Society of Japan, September 25, 2012 have been attempted; however, it is not clear how effective these methods are. An object of the present invention is to provide a method of producing a glycoprotein having a desired complex-type sugar chain which is efficient and inexpensive as compared to conventional methods.

### SUMMARY OF THE INVENTION

The present inventors have dedicated to repetitive studies to achieve the above object. As a result, they have unexpectedly found that the antibody having inhibitory ability to N-acetylglucosaminidase activity is expressed in an insect, whereby a glycoprotein having a desired complex-type sugar chain can be produced efficiently and inexpensively, and have completed the present invention.

The present invention provides a method of producing a glycoprotein. The method comprises the steps of: introducing into an insect organism or insect cells a gene encoding a desired protein and a gene encoding an antibody that inhibits a decomposing enzyme preventing formation of a desired complex-type sugar chain in the desired protein; and obtaining a desired protein having a desired complex-type sugar chain from the insect organism or insect cells obtained in the introduction step.

In the method, the decomposing enzyme may be a membrane protein acting in cells. The decomposing enzyme may be an N-acetylglucosaminidase. The desired complex-type sugar chain may be a complex-type sugar chain with galactose or sialic acid linked to at least one non-reducing end. The method may further comprise the step of introducing at least one selected from the group consisting of a gene encoding galactose transferase and a gene encoding sialyltransferase into the insect organism or insect cells. The method may further comprise the step of introducing at least one selected from the group consisting of sialic acid and a sialic acid derivative into the insect organism or insect cells. The method may further comprise the step of administering at least one selected from the group consisting of deoxygalactonojirimycin, methyl β-galactopyranoside and lactose into the insect organism or insect cells. At least one selected from the group consisting of the gene encoding a desired protein and the gene encoding an antibody may be inserted into at least one vector. The insect may be a lepidopteran insect. The insect may be Bombyx mori.

One aspect of the present invention is a vector comprising a gene encoding an antibody that inhibits activity of a membrane protein suppressing formation of a desired sugar chain in a desired protein.

One aspect of the present invention is a kit. The kit comprises: a vector comprising a gene encoding an antibody that inhibits activity of a membrane protein suppressing formation of a desired sugar chain in a desired protein; and a vector comprising a gene encoding a desired protein.

One aspect of the present invention is an insect organism or insect cell into which a vector comprising a gene encoding an antibody and a vector comprising a gene encoding a desired protein are introduced. The antibody inhibits activity of a membrane protein suppressing formation of a desired sugar chain in a desired protein.

### BRIEF DESCRIPTION OF THE DRAWINGS

Fig. 1 is a view showing N-acetylglucosaminidase inhibitory activities of BmFDL inhibitory antibody clones.
Fig. 2 is a view showing N-acetylglucosaminidase inhibitory activities of recombinant BmFDL antibodies.
Fig. 3 is a graph illustrating the measurement results of the amount of sialic acid added.
Fig. 4 is a graph illustrating the measurement results of the amount of galactose added.

### DETAILED DESCRIPTION OF THE PREFERRED EMBODIMENTS

In the methods of producing a glycoprotein envisaged herein (hereinafter, simply referred to as "production method"), a gene encoding a desired protein and a gene encoding an antibody that inhibits a decomposing enzyme preventing formation of a desired complex-type sugar chain in the desired protein are introduced into an insect organism or insect cells.

The insect is not particularly limited as long as it is an insect (Insecta) suitable for expressing recombinant proteins. The insect includes preferably a lepidoptera insect, more preferably a lepidopteran insect selected from the group consisting of Bombycidae, Nocuidae, Arctiidae, and Saturniidae, and the like. More specifically, the species thereof include Bombyx mori, Spilosoma imparilis, Antheraea pernyi, Spodoptera frugiperda, and Trichoplusiani, and the like. Among them, Bombyx mori is particularly preferred.

The insect organism may be at any stage of imago, pupa, and larva. The stage of pupa or larva is preferred. From the viewpoint of serine protease activity and the sensitivity to baculovirus, it is particularly preferable to use a pupa of the insect organism.

The insect cells are not particularly limited as long as they are cell lines established from insects suitable for expressing recombinant proteins. Examples thereof include BmN, BmN4, SpIm, Anpe, Sf9, Sf21, High5, and S2.

The desired protein is not particularly limited as long as it is a protein to which a sugar chain may be added. In this embodiment, alkaline phosphatase (ALP) or the like may be used.

The decomposing enzyme preventing formation of a desired sugar chain in the desired protein is not particularly limited as long as it is known to a person skilled in the art. The decomposing enzyme may be preferably a decomposing enzyme as a membrane protein acting in cells, more preferably N-acetylglucosaminidase, still more preferably β-N-acetylglucosaminidase involved in decomposition of chitooligosaccharides (chitooligosaccharidolytic β-N-acetylglucosaminidase; AgHEX02, DmHEX02, BmHEX01, TnHEX01, SfHEXA, SfHEXB, SfHEXB1, etc.), β-N-acetylglucosaminidase involved in N-glycan processing (N-glycan processing β-N-acetylglucosaminidase; BmFDL, DmFDL, SfFDL, AmFDL, GmFDL, SfFDL, etc.), BmGlcNAcase, BmGlcNAcase1, BmGlcNAcase2 or the like. Among them, BmFDL is particularly preferred.

The antibody that inhibits a decomposing enzyme preventing formation of a desired sugar chain in the desired protein is not particularly limited, and appropriately designed by a person skilled in the art based on the decomposing enzyme to be inhibited. In this embodiment, an antibody that inhibits N-acetylglucosaminidase activity may be used. Examples of the antibody include antibodies each having an IgG1 heavy-chain (γ chain) constant region whose nucleic-acid base sequence is known under Accession No: P01868 and a κ chain constant region whose nucleic-acid base sequence is known under Accession No: P01837.

The above antibody can be produced, for example, in the following manner. First, an antigen site of the decomposing enzyme to be inhibited is selected, and then an animal such as a mouse is immunized with the antigen. The immunization can be carried out by any method known to a person skilled in the art. The antigen may be mixed with an adjuvant such as Freund's complete adjuvant or Freund's incomplete adjuvant. After the final immunization, the spleen is extracted from the immunized animal, and spleen cells are separated to obtain antibody-producing cells. Then, the obtained antibody-producing cells are fused with other cells such as myeloma cells to form hybridomas. Monoclonal antibodies produced from the obtained hybridomas are purified. The inhibitory activities of the monoclonal antibodies are measured, and a gene of the antibody having an inhibitory activity is cloned in a vector for insects. The antibody is produced in an insect and purified, and the inhibitory activity of the antibody is confirmed. A series of these steps can be carried out by any method known to a person skilled in the art.

A gene encoding the above antibody and a gene encoding a desired protein can be introduced into an insect by any method known to a person skilled in the art. Examples of the method include methods of using vectors such as viruses, plasmids, cosmids or fosmids. Among them, the method of using a viral vector is preferred, and the method of using a baculovirus vector is particularly preferred. Specific examples of the baculovirus include BmNPV, AcNPV, HycuNPV, and AnpeNPV. In this embodiment, the above genes may be introduced into an insect using the baculovirus vector. The vector may be introduced so that the introduced genes can be transiently expressed, or so that the introduced genes can be continuously expressed by a transgenic technology or the like. When the above antibody is expressed using a baculovirus vector in this manner, the virus is being amplified and the antibody is being expressed as long as the insect remains alive. Thus, the decomposing enzyme can be inhibited more efficiently as compared to the case of administering a compound.

At least one of a gene encoding galactose transferase, a gene encoding an N-acetyl glucosamine transferase, a gene encoding sialyltransferase, and a gene encoding fucosyltransferase may be introduced into the insect.

The galactose transferase is not particularly limited as long as it is an enzyme which can transfer galactose from a sugar donor to a sugar chain. The galactose transferase is preferably a β-1,4-galactose transferase, more preferably a β-1,4-galactose transferase I (GalT I), β-1,4-galactose transferase II (GalT II), β-1,4-galactose transferase III (GalT III), β-1,4-galactose transferase IV (GalT IV) or the like. The origin of the galactose transferase is not particularly limited, but the galactose transferase is preferably derived from mammals. In this embodiment, GalT III derived from mice (Mus musculus) (mGalT III) may be used.

The sialyltransferase is not particularly limited as long as it is an enzyme which can transfer sialic acid from a sugar donor to a sugar chain, but it is preferably α2, 3-sialyltransferase, α2, 6-sialyltransferase or the like. Particularly preferably, the sialyltransferase adds sialic acid to the galactose at the non-reducing end of a complex-type sugar chain in an α-2,3 linkage or α-2,6 linkage. The origin of the sialyltransferase is not particularly limited, but the sialyltransferase is preferably derived from mammals. In this embodiment, hST derived from humans may be used.

The N-acetyl glucosamine transferase is not particularly limited as long as it is an enzyme which can transfer N-acetyl glucosamine from a sugar donor to a sugar chain. Preferably, the N-acetyl glucosamine transferase is an N-acetyl glucosamine transferase I (GnT I), II (GnT II) or the like. Particularly preferably, GnT I is used singly, or GnT I and GnT II are used in combination. Each gene encoding each of the N-acetyl glucosamine transferases III, IV, V, and VI (Gnt III to VI) is introduced into an insect, whereby a bisecting complex-type sugar chain or a tri- to penta-antennary complex-type sugar chain can be also obtained. The origin of the N-acetyl glucosamine transferase is not particularly limited, but the N-acetyl glucosamine transferase may be preferably derived from silkworms or mammals.

The fucosyltransferase is not particularly limited as long as it is an enzyme which can transfer fucose from a sugar donor to a sugar chain. Specific examples thereof include α1, 3-fucosyltransferase and α1,6-fucosyltransferase. Particularly preferably, the transferase adds fucose to the N-acetyl glucosamine at the reducing end of a complex-type sugar chain in an α-1,6 linkage. The origin of the fucosyltransferase is not particularly limited, but the fucosyltransferase is preferably derived from silkworms or mammals.

The production method may include the step of administering at least one of sialic acid, a sialic acid derivative and a precursor into an insect organism or insect cells. Examples of the sialic acid derivative and the precursor include CMP-Neu5Ac, Neu5Ac, Neu5Ac-9-P, ManNAc-6-P, and UDP-GlcNAc.

The production method may include the step of administering at least one of deoxygalactonojirimycin, methyl β-galactopyranoside and lactose into an insect organism or insect cells.

The route of administration of at least one of the above substances is not particularly limited, and may be injection administration, oral administration, application, or the like.

A dose of at least one of the above substances is not particularly limited, but is preferably 0.10 mg or more per treatment.

The dosing period and dosing interval of at least one of the above substances are not particularly limited. For example, the substance may be administered for one week, once a day or once every two days.

An enzyme necessary to synthesize a sialic acid derivative and a precursor, such as CMP-Nue5Ac synthetase, Neu5Ac-phosphate synthetase, Neu5Ac9-phosphate synthetase or UDP-GlcNAc2 epimerase/ManNAc kinase, may be further introduced into the insect organism or insect cells.

In the production methods envisaged herein, a desired protein having a desired complex-type sugar chain formed is obtained from the insect organism or insect cells obtained in the introduction step.

In general, sugar chains are roughly classified into the following categories: O-linked sugar chains and N-linked sugar chains. The term "O-linked sugar chain" refers to a sugar chain that is linked to a hydroxy group of a serine or threonine residue of a protein or polypeptides via an O-glycosidic linkage. Specific examples thereof include, but are not limited to, sugar chains including a sugar chain structure represented by any one of Formulae (12) to (16) below:

[Chemical Formula 1 ] Gal β 1 - 3GalNAc···(12) GlcNAc β 1 - 3GalNAc···(13) GalNAc β 1 **-** 3GalNAc···(14) GlcNAc β 1 - 6GalNAc···(15) GalNAc α 1 - 6GalNAc··· (16)

GlcNAc may be linked to N-acetyl galactosamine (GalNAc) at the reducing end side. The term "reducing end side" refers to a GalNAc side that is linked to a serine or threonine residue of a protein or polypeptides. In Formulae (12) to (16) above, the right side corresponds to the reducing end side. On the other hand, the opposite side to the GalNAc side that is linked to a serine or threonine residue (this corresponds to the left side in Formulae (12) to (16) above) is referred to as the "non-reducing end". Hereinafter, when sugar chain structures are represented by the same formulae, the right side is described as the "reducing end side" and the left side is described as the "non-reducing end side". The above formulae also show sugar-binding forms. For example, when "β1-3" is described between two sugars, these sugars are linked to each other through a β1,3 linkage at 1-position of the sugar at the left side and 3-position of the sugar at the right side. In the above formulae, Gal represents galactose, and GlcNAc represents N-acetyl glucosamine.

The term "N-linked sugar chain" refers to a sugar chain that is linked to an amino group of an asparagine residue of a protein or polypeptides via an N glycosidic linkage. In general, the N-linked sugar chain is roughly classified into the following categories: pauci-mannose-, high-mannose-, hybrid-. or complex-type N-linked sugar chain.

In this embodiment, a pauci-mannose-type sugar chain refers to (i) a sugar chain in which mannose is β-1,4-linked to N-acetyl glucosamine (GlcNAc) at the non-reducing end side in a diacetyl chitobiose moiety at the reducing end of the N-linked sugar chain; and (ii) a sugar chain in which one or two mannoses are β-1,3-linked and/or β-1,6-linked to the mannose at the non-reducing end side in the sugar chain of (i). Specifically, it may be a sugar chain having a sugar chain structure represented by any one of Formulae (4) to (7) below: 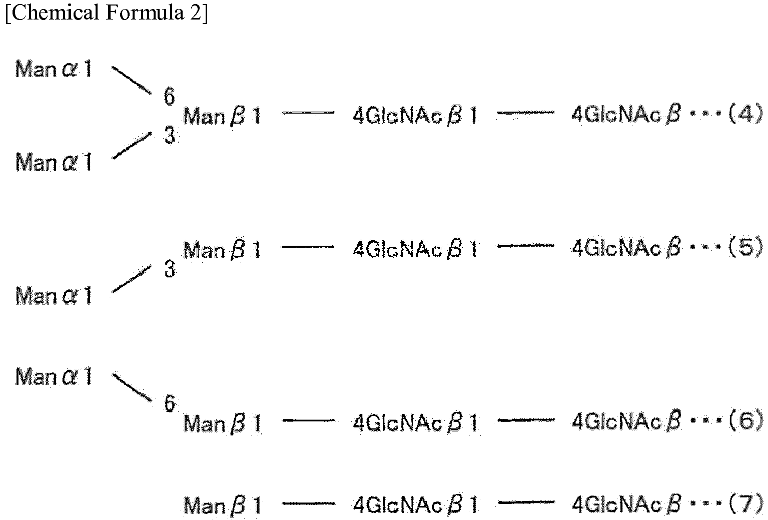 In these formulae, the right side is described as the "reducing end side" and the left side is described as the "non-reducing end side". In the above formulae, Man represents mannose. In the specification, a sugar chain having a sugar chain structure represented by Formula (4) is also referred to as a "mannose-core-type sugar chain".

In this embodiment, the term "high-mannose-type sugar chain" refers to a sugar chain in which one or more mannoses (no sugars other than mannose) are further linked to the mannose having the sugar chain structure of Formula (4) at the non-reducing end side. Examples thereof include sugar chains in which one or more mannoses (no sugars other than mannose) are further linked to the mannose having a sugar chain structure represented by Formula (8) below: 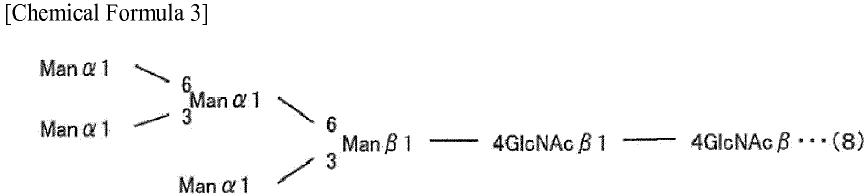 at the non-reducing end side. More specific examples thereof include sugar chains including a sugar chain structure represented by Formula (9) below:

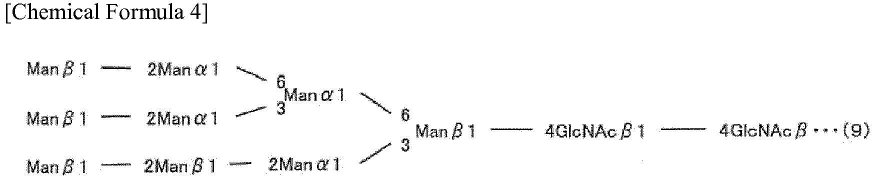

In this embodiment, the term "hybrid-type sugar chain" refers to a sugar chain in which a sugar other than mannose is linked to one mannose having a sugar chain structure of Formula (4) below: 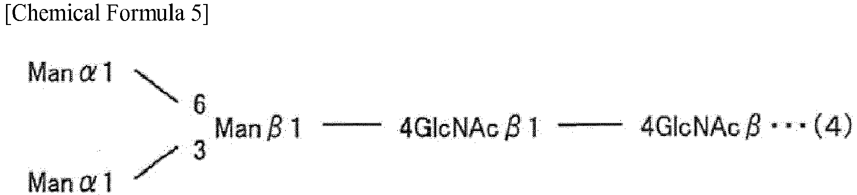 at the non-reducing end side and one or more mannoses (no sugars other than mannose) are further linked to the other mannose. Examples thereof include sugar chains having a sugar chain structure represented by Formula (10) below: 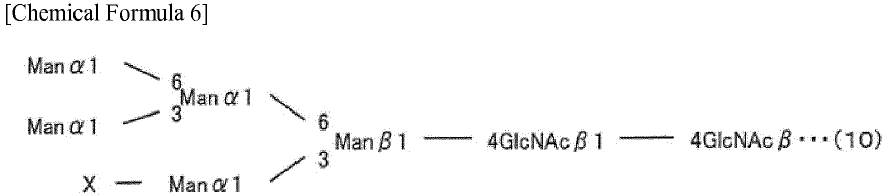 (wherein X represents one or more sugars other than mannose and may be present in the plural). More specific examples thereof include sugar chains including a sugar chain structure represented by Formula (11) below:

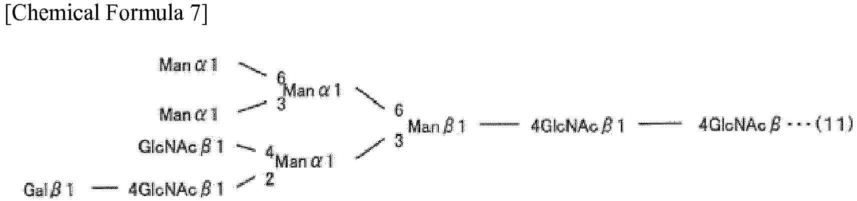

The hybrid-type sugar chain may be a sugar chain in which fucose is linked, particularly α-1,6-linked to N-acetyl glucosamine at the reducing end side in the diacetyl chitobiose moiety. The hybrid-type sugar chain may be a sugar chain in which one or more N-acetyl glucosamines are further linked, particularly β-1,4-linked to the mannose β-1,4 linked to the diacetyl chitobiose moiety (so-called bisecting sugar chain).

In this embodiment, the term "complex-type sugar chain" refers to
(i) a sugar chain that includes a sugar chain structure represented by Formula (1) or (2) below: 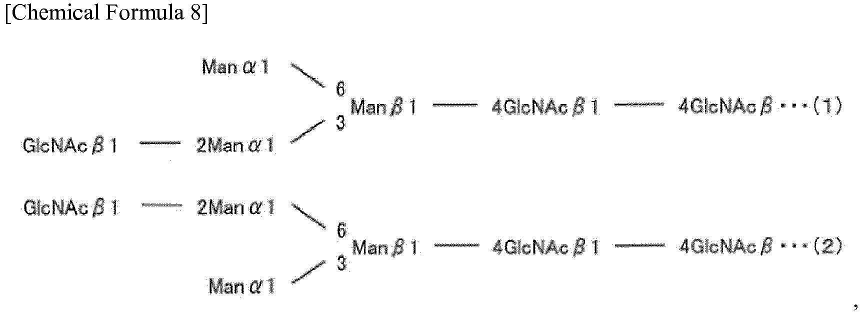 and includes a sugar chain structure in which no mannose is linked to the mannose at the non-reducing end side to which N-acetyl glucosamine is not linked; and
(ii) a sugar chain including a sugar chain structure of Formula (3): 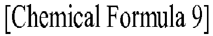 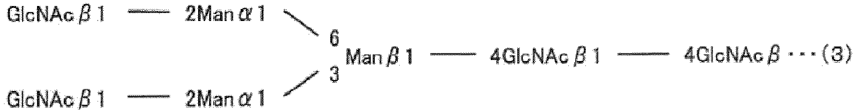
The sugar chain structure of Formula (3) may encompass, for example, sugar chains including sugar chain structures represented by Formulae (17) to (20) below: 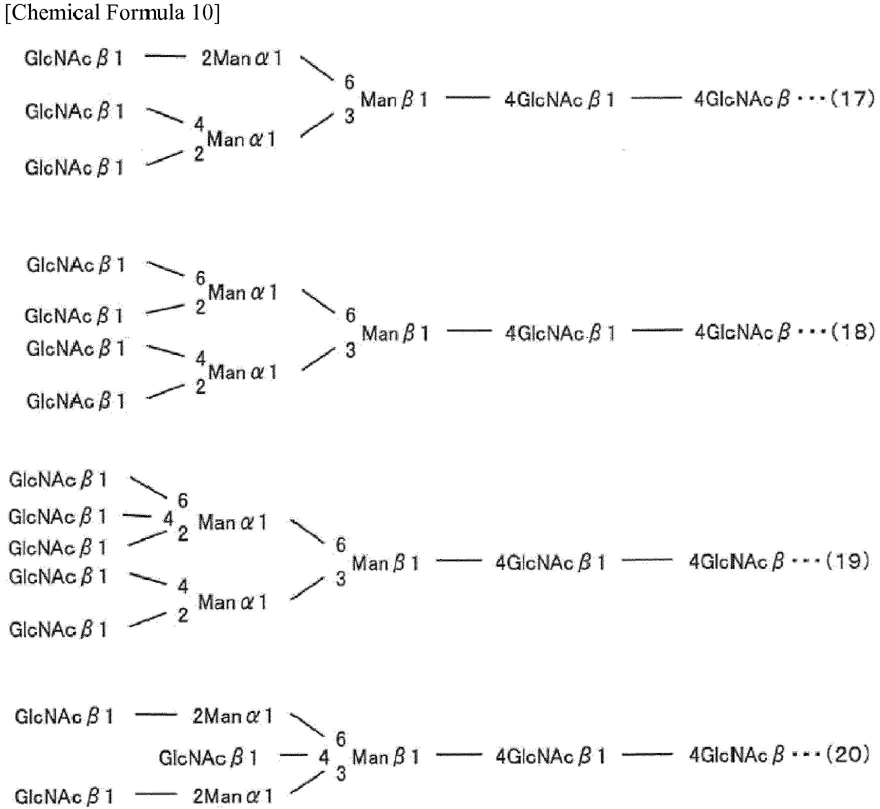 The sugar chain structures of Formulae (17) to (19) are so-called triantennary, tetraantennary, and pentaantennary structures, respectively. The sugar chain structure of Formula (20) is a so-called bisecting sugar chain.

The complex-type sugar chain may be a sugar chain in which fucose is linked, particularly α-1,6-linked to N-acetyl glucosamine at the reducing end side in the diacetyl chitobiose moiety. The complex-type sugar chain may be a sugar chain in which one or more N-acetyl glucosamines are further linked, particularly β-1,4-linked to the mannose β-1,4 linked to the diacetyl chitobiose moiety (so-called bisecting sugar chain).

The glycoprotein having a desired complex-type sugar chain which is obtained by the production method is not particularly limited as long as it is a glycoprotein obtained by inhibiting the decomposing enzyme activity using the antibody. Preferably, the glycoprotein may be a protein having a complex-type N-linked sugar chain with N-acetyl glucosamine, galactose or sialic acid at the non-reducing end. Examples of the sugar chain include:
(i) complex-type sugar chains including sugar chain structures represented by Formulae (1) to (3) below: 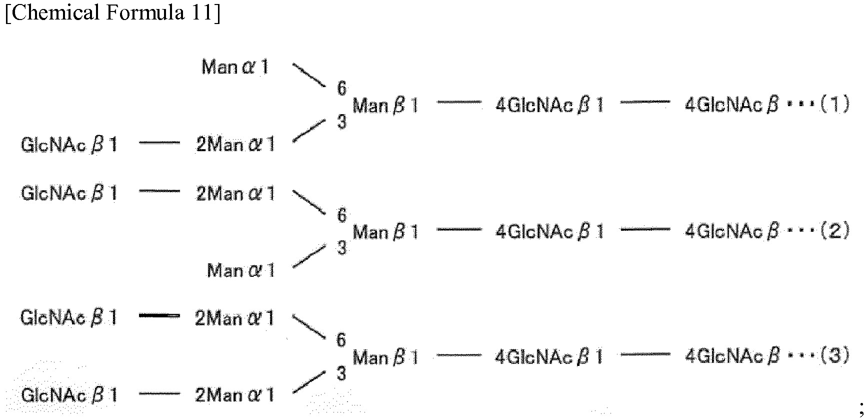
(ii) complex-type sugar chains including sugar chain structures represented by Formulae (21) to (25) below: 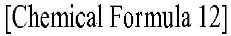 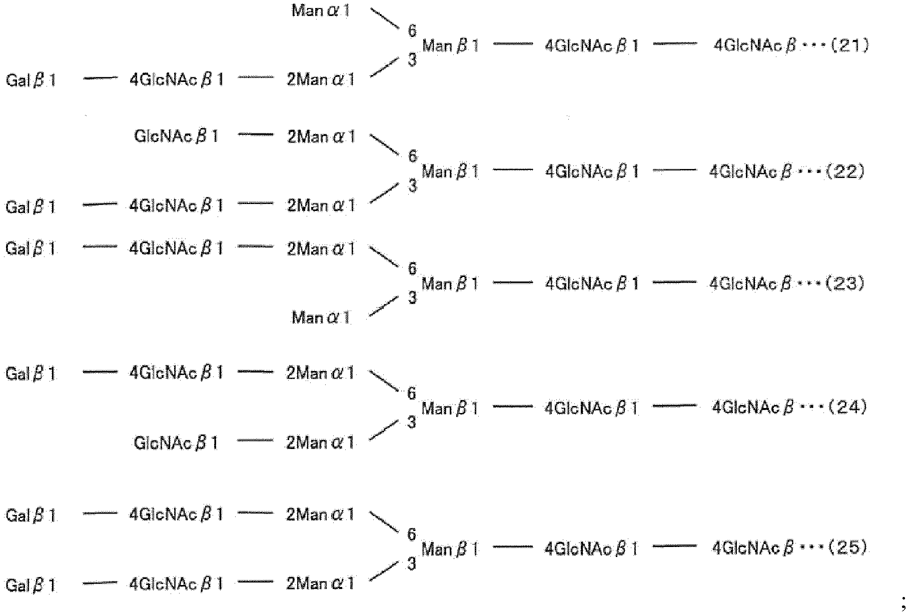 and
(iii) complex-type sugar chains in which one or more sialic acids are α-2,3-linked and/or α-2,6-linked to the galactose having any one of the sugar chain structures represented by Formulae (21) to (25) above at the non-reducing end side.

The desired complex-type sugar chain may be a sugar chain in which fucose is linked, particularly α-1,6-linked to N-acetyl glucosamine at the reducing end side in the diacetyl chitobiose moiety. The desired complex-type sugar chain may be a sugar chain in which one or more N-acetyl glucosamines are further linked, particularly β-1,4-linked to the mannose β-1,4 linked to the diacetyl chitobiose moiety (so-called bisecting sugar chain).

In this embodiment, the N-acetylglucosaminidase activity in an insect organism into which a galactose transferase and/or sialyltransferase is introduced is inhibited using the antibody that inhibits the N-acetylglucosaminidase activity, whereby a complex-type sugar chain with galactose or sialic acid at the non-reducing end can be produced.

The resulting glycoprotein having a desired complex-type sugar chain can be obtained using any method known to a person skilled in the art. For example, an insect organism is ground by any method known to a person skilled in the art and subjected to ultracentrifugal separation, whereby a target protein can be collected. The glycoprotein obtained as described above may be further purified by any method known to a person skilled in the art, for example, chromatography.

The scope of the present disclosure encompasses a vector containing a gene encoding an antibody that inhibits membrane protein activity by which formation of a desired sugar chain in a desired protein is suppressed. The vector may be in the form of a kit or may be introduced into an insect organism or insect cells. The scope of the present disclosure includes the kit and the insect organism or insect cells.

Hereinafter, the present disclosure will be described in detail with reference to examples, however the present disclosure is not limited to the examples.

### EXAMPLES

### Reference Example 1: Preparation of BmFDL Inhibitory Antibody

### Production of Recombinant BmFDL

Regarding the production of an antigen (recombinant BmFDL) used for immunization of mice, mRNA was extracted from silkworm larvae using the MagExtractor mRNA isolation kit (manufactured by TOYOBO CO., LTD.). Primers having the base sequences represented by SEQ ID NOs: 12 and 13 were produced, and 5' and 3' terminal regions of the BmFDL gene were amplified using the BD Advantage 2 polymerase system. The DNA fragment amplified was inserted into the pT7-Blue T-vector (manufactured by TAKARA BIO INC.), and sequence analysis was performed using the Big Dye sequencing reagent (Applied Biosystems, Foster City, CA, USA) and primers having the base sequences represented by SEQ ID NOs: 14 and 15.

The full length BmFDL cDNA fragment was amplified using a primer set of SEQ ID NOs: 16 and 17, treated with BglII and XhoI restriction enzymes, and cloned to the baculovirus transfer vector pM01 (manufactured by Sysmex Corporation). Silkworm cells were co-transfected with the resulting viral vector and B. mori nucleopolyhedrovirus DNA. The resulting recombinant viruses were screened by limiting dilution using a 96-well plate. Then, a purified BmFDL-expressing recombinant baculovirus was infected with a silkworm, and the body fluid was collected at the end stage of infection. Forty ml of the silkworm body fluid was diluted with 160 ml of 100 mM Tris buffer (pH 8.0) containing 150 mM NaCl (TBS). The diluted body fluid was applied to the Strep-Tactin Superflow column (IBA) which had been equilibrated with TBS. The washed column was eluted with TBS containing 200 ml of 2.5 mM desthiobiotin, and the eluate containing dissolved proteins was recovered. After that, the eluted fraction was 10-fold diluted with DW and the diluted solution was applied to the HiTrap Q HP column (GE healthcare) which had been equilibrated with 20 mM Tris buffer (pH 8.0). The column was washed with Tris buffer (pH 8.0), and subjected to gradient elution from 0 M NaCl to 1.0 M NaCl. The fraction of protein adsorbed was collected and used as a purified substance.

### Immunization of Mice

The recombinant BmFDL (50 µg) obtained in the above manner was mixed with a complete-adjuvant TiterMax Gold (TiterMax USA, Inc.). The resulting mixture was injected into foot-pads of mice for primary immunization. Two weeks after the primary immunization, the mixture was injected into foot-pads of mice for additional immunization. Two days before the hybridomas were prepared (described below), only BmFDL was intravenously injected for final immunization.

### Cell Fusion

Two days after the final immunization, the spleen was collected from each mouse and mixed with mouse myeloma cells P3/X63-AG8.653 at a ratio of 5 : 1. Cell fusion was performed using a fusion agent such as polyethylene glycol 1500 (Roche Diagnostics, Inc.). Then, the fused cells were subjected to drug selection in the RPMI1640 culture medium containing HAT (manufactured by Sigma) and 10% FCS to obtain many hybridomas.

### Screening of Hybridoma

### Primary Screening ELISA

An anti-BmFDL monoclonal antibody-producing hybridoma was screened from the hybridomas obtained in the above manner. Specifically, 0.05 µg of antigen (recombinant BmFDL) was first immobilized on a 96-well plate (Nunc immunoplate Maxisoap) at room temperature for 1 hour. The plate was washed 3 times with PBS, and 1% BSA/PBS was immobilized at room temperature for 1 hour, so that blocking was performed. Thereafter, the resulting product was washed 3 times with TPBS, and incubated together with the supernatants of the hybridoma cultures 2-fold diluted with 1% BSA/TPBS at room temperature for 1 hour. The resulting product was washed 3 times with TPBS, and incubated together with HRP-labeled anti-mouse IgG antibody (10,000-fold dilution) at room temperature for 1 hour, followed by washing 3 times with TPBS. HRP was detected. As a result, a plurality of the anti-BmFDL monoclonal antibody-producing hybridomas was selected.

### Secondary Screening Immunoprecipitation

Protein G sepharose beads (30 µl) were placed into a microtube. Then, 500 µl of the supernatants of the hybridoma cultures 2-fold diluted with 1% BSA/TPBS was added and rotated at 4°C overnight, and then 1 mg of antigen was added and rotated at 4°C for 1 hour. The resulting mixture was washed 3 times with TPBS. A sample buffer for SDS-PAGE (30 mL) was added and the resulting mixture was boiled at 100°C for 5 minutes, followed by SDS-PAGE. As a result, eight anti-BmFDL monoclonal antibody-producing hybridomas were selected.

### Purification of Antibodies

Antibodies were purified from the supernatants of the eight hybridoma cultures using ProteinG (GE healthcare), and the neutralization activity was measured. Specifically, the neutralization activity was measured in the following manner.

### Measurement of Neutralization Activity (Search of BmFDL Inhibitory Antibody)

Each of the BmFDL monoclonal antibody clones (10 µl) shown in Table 1 below was allowed to react with 1 µl of 0.5 mg/ml purified BmFDL at 4°C for 1 hour.

**[Table 1]**

| Clone NO. | Antibody concentration |
|---|---|
| 1-96-21 | 1.68 mg/ml |
| 1-96-9 | 1.91 mg/ml |
| 1-57-4 | 1.12 mg/ml |
| 1-201-2 | 2.18 mg/ml |
| 1-406-4 | 1.75 mg/ml |
| 1-57-2 | 1.27 mg/ml |
| 1-404-1 | 2.24 mg/ml |
| 1-99-4 | 3.76 mg/ml |

Then, 10 µl of a 1M phosphate-citrate buffer, 2 µl of PA-sugar chain substrate: PA-Sugar Chain 012 (Takara), 2 µl of 20% Triton X-100, 75 µl of DW were added to each of the samples, and the resulting mixtures were allowed to react at 30°C for 16 hours under stirring. The resulting samples were heated at 98°C for 3 minutes and cooled on ice. The samples were subjected to centrifugation at 12,000 rpm for 5 minutes and the supernatants of the samples were analyzed by HPLC. The following materials were used for HPLC analysis. A solvent A (DW), a solvent B (20% acetonitrile), and a solvent C (0.2% TFA) were mixed at a ratio of A : B : C = 90 : 0 : 10, and the resulting mixture was passed through the column (Cosmosil 5C18-AR2 (nacalai tesque)) at a flow rate of 1.2 ml/min for equilibration. The column temperature was 35°C.

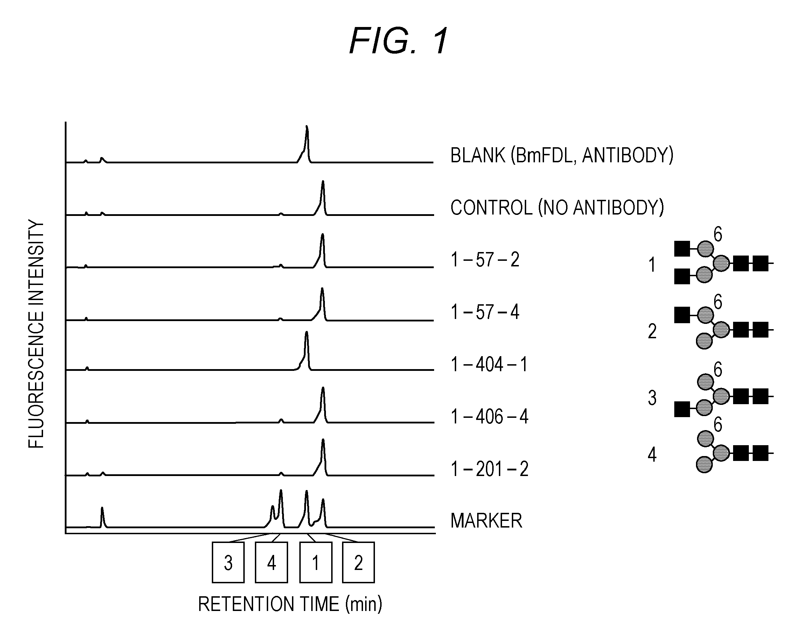

The supernatant for HPLC analysis (10 µl) was injected and the gradient elution from 0% to 20% B and from 90% to 70% A was performed for 5 to 40 minutes. The PA-sugar chain was detected by fluorescence (Ex = 310 nm and Em = 380 nm). As the result of the experiment, the inhibition of BmFDL activity was observed in only the clone 1-404-1, and the antibody of this clone was shown to have neutralization activity (Fig. 1).

### Reference Example 2: Obtainment of Inhibitory Antibody Gene

The gene of the antibody of the clone 1-404-1 having a neutralization activity was obtained from the hybridomas. First, the hybridomas were cultured, and mRNA was purified from the cultured hybridomas in accordance with the MagExtractor mRNA protocol. Next, 5'RACE was performed in accordance with the SMARTer RACE cDNA kit protocol, and the gene sequence of the antibody variable region was determined. The primers shown in Table 2 below were prepared from the determined sequence and cloned to the gene cloning vector (Sysmex Corporation). The resulting sequence was confirmed using the DNA sequencer (manufactured by ABI).

**[Table 2]**

| Antibody gene | Primer 1 | SE Q ID NO: | Primer 2 | SEQ ID NO: | Vector for cloning |
|---|---|---|---|---|---|
| γ chain | gaGCGGCCGCATGGCTTG GGTGTGGACCTTG | 18 | TGAGGAGACTGTG AGAGTGGTG | 19 | pCasMmG1 C |
| κ chain | ggAGATCTATGGAGACAG ACACACTCCTGC | 20 | | | pCasMmK C |

| | | | | | |
|---|---|---|---|---|---|
| Antibody constant region MmG1C: murine IgG1 heavy chain constant region Accession No: P01868 Antibody constant region MmKC: murine κ chain constant region Accession No: P01837 | | | | | |

### Reference Example 3: Expression of Recombinant BmFDL Activity Inhibitory Antibody in Silkworms

### Expression of Antibody in Baculovirus

Amplified products were obtained from the gene sequence obtained in the above manner by PCR using each of the primers having the base sequences represented by SEQ ID NOs: 1 to 4 (refer to Table 3 below). The amplified products were subjected to restriction enzyme treatment with various restriction enzymes shown in Table 4 below and ligated to the transfer vectors (Sysmex Corporation) shown in Table 4 below which had been similarly treated with restriction enzymes and dephosphorylated. Competent cells DH5α were transformed by the calcium chloride method, and thus the transfer vectors of the genes were obtained. The sequences inserted into the transfer vectors were analyzed using the DNA sequencer (manufactured by ABI).

**[Table 3]**

| Enzyme | Primer 1 | SEQ ID NO: | Primer 2 | SEQ ID NO: |
|---|---|---|---|---|
| BmFDL inhibitory antibody (γ chain) | 5'                                                    - ccagatcttatgagcactgaacacagacacctc -3' | 1 | 5'                                                    - gggatatcctatttaccaggagagtgggagaggc -3' | 2 |
| BmFDL inhibitory antibody (κ chain) | 5' - ggcagatcttatggttttcacacctcagatac -3' | 3 | 5'- ccgatatcctaacactcattcctgttgaagc -3' | 4 |
| ALP | 5'- caggtaccggtacctaggtaaaacaaaatgc | 5 | 5' - ggtctagagtcagacaagcctgcagggg -3' | 6 |
| | -3' | | | |
| mGalT3 | 5'- gaggatatcatgttgcggaggctgctggag -3' | 7 | 5’ - gactctagattagtgtgagccacggggagctg -3’ | 8 |
| hST | 5'                                    - catcgagctcatgattcacaccaacctgaag -3' | 9 | 5’ - ctgtctagagcagtgaatggtccggaagccag -3’ | 10 |

**[Table 4]**

| Enzyme | Restriction enzyme at N-end side | Restriction enzyme at C-end side | Transfer vector |
|---|---|---|---|
| BmFDL Inhibitory Antibody (γ chain) | BglII | EcoRV | pCPM |
| BmFDL Inhibitory Antibody (κ chain) | BglII | EcoRV | pM |
| ALP | KpnI | Xba I | pM31 |
| mGalT3 | Sma I | Xba I | pV01 |
| hST | Sac I | Xba I | pM31 |

Production of recombinant baculovirus was performed by co-transfecting DNA of linear ABvNPV (Sysmex Corporation) with the transfer vectors. Specifically, to prepare the stationary culture, BmN cells were plated to a 35 mm cell culture dish to form a monolayer of about 5x10⁵ cells. Linear ABv baculovirus DNA (0.2 µg) (Sysmex Corporation), 0.5 µg of each of the baculovirus transfer vectors having foreign genes inserted, and 100 µl of TC-100 (non-serum) were placed in a 1.5 ml tube and mixed therein, followed by being allowed to stand at room temperature for 15 minutes. TC-100 (non-serum) (100 µl) was mixed with 8 µl of a cationic lipid reagent (X-tremeGENE HP DNA Transfection Reagent; Roche), followed by being allowed to stand at room temperature for 15 minutes. Then, the two solutions were mixed and the resulting mixture was allowed to stand at room temperature for 15 minutes. After that, 800 µl of TC-100 (non-serum) was added thereto. This mixture solution was added to the monolayer of BmN cells in the 35 mm cell culture dish and cultured at 25°C for 16 hours. Thereafter, the mixture was removed and 2 ml of TC-100 (containing 10% FBS) was newly added, followed by stationary culture at 25°C for seven days. The resulting culture supernatant was used as a recombinant virus stock solution.

In order to isolate the recombinant ABv baculovirus having foreign genes inserted from the recombinant virus stock solution, BmN cells were first placed in each well of 96-well plates (amount per well: 1.5 x 10⁴ cells/50 µl TC-100 (10% FBS)) and cultured. The recombinant virus stock solution was diluted to six concentrations: 10⁻⁴, 10⁻⁵, 10⁻⁶, 10⁻⁷, 10⁻⁸, and 10⁻⁹ using TC-100 (10% FBS), and each of the diluted solutions was added to each well of the plates (amount per well: 50µl), resulting in infection of the cells. These plates were sealed to avoid dryness and subjected to stationary culture at 25°C. The recombinant virus was selected by confirming infection symptoms on 7th day after infection under optical microscope. Each of the culture supernatants was used as an isolated virus solution "BmFDL inhibitory antibody-NPV".

### Expression of Recombinant Inhibitory Antibody Using Silkworm

In order to confirm the recombinant inhibitory antibody activity, BmFDL inhibitory antibody-NPV was inoculated into 5th instar 1st day silkworm larvae (Kinshu Showa), and the body fluid of the silkworm larvae (5th instar 7th day) was sampled.

### Purification of Inhibitory Antibodies Expressed Using Silkworms

Expressed inhibitory antibodies were purified using ProteinG (GE healthcare). The purified antibodies were subjected to protein quantification at an absorbance of 280 nm.

### Reference Example 4: Measurement of Neutralization Activity of Recombinant Inhibitory Antibody

### Measurement of Neutralization Activity (Evaluation of Neutralization Activity of Baculovirus Expressing BmFDL Antibody (Strain 1-404-1))

Purified BmFDL at 0.5 mg/ml (1 µl) and 17.6 or 44.4 µg of purified anti-BmFDL monoclonal antibody (clone 1-404-1) or purified recombinant BmFDL antibody using the sequence of this clone were reacted at 4°C for 1 hour. The components shown in Table 5 below are added to the samples.

**[Table 5]**

| | Substrate (PA-sugar chain) | BmFDL antibody | Enzyme (BmFDL) |
|---|---|---|---|
| Blank | ○ | - | - |
| Control | ○ | - | ○ |
| Monoclonal antibody | ○ | ○ | ○ |
| Recombinant antibody | ○ | ○ (Recombinant antibody) | ○ |

First, 10 µl of a 1M phosphate-citrate buffer, 2 µl of PA-sugar chain substrate: PA-Sugar Chain 012 (Takara), 2 µl of 20% Triton X-100, and 2 µl of DW were added to each of the samples to have a total volume of 100 µl. The resulting mixtures were stirred and reacted at 30°C for 16 hours. Then, the resulting samples were heated at 98°C for 3 minutes and cooled on ice. The samples were subjected to centrifugation at 12,000 rpm for 5 minutes and the supernatants of the samples were analyzed by HPLC. The used column was Cosmosil 5C18-AR2 (nacalai tesque). A solvent A (DW), a solvent B (20% acetonitrile), and a solvent C (0.2% TFA) were mixed at a ratio of A : B : C = 90 : 0 : 10, and the resulting mixture was passed through the column at a flow rate of 1.2 ml/min for equilibration. The column temperature was 35°C. The sample for analysis (10 µl) was injected and the gradient elution from 0% to 20% B and from 90% to 70% A was performed for 5 to 40 minutes. The PA-sugar chain was detected by fluorescence (Ex = 310 nm and Em = 380 nm).

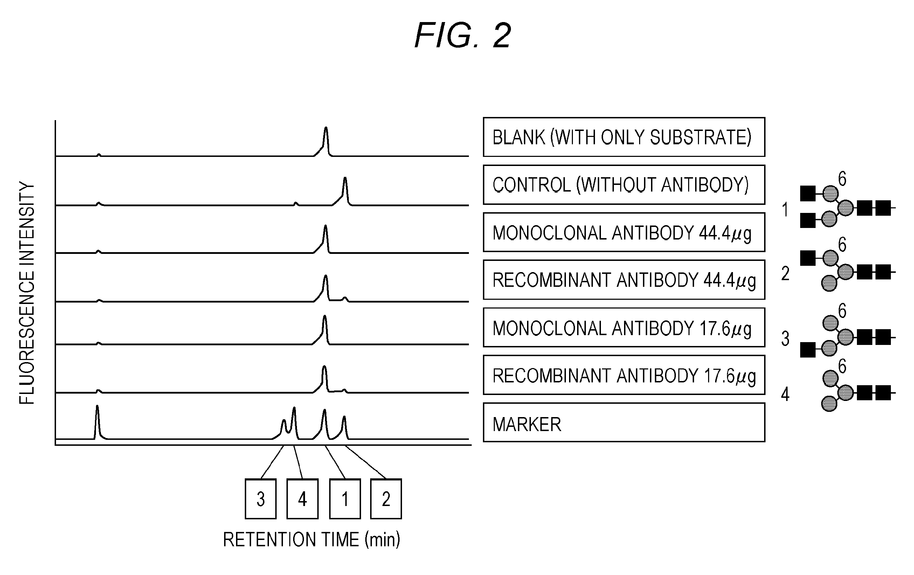

As a result, although the inhibition effect was slightly lower than that of a natural monoclonal antibody, the inhibition effect of GlcNAcase (BmFDL) was observed even in the recombinant BmFDL antibody. This shows that the recombinant antibody has neutralization activity (Fig. 2).

### Reference Example 5: Production of Recombinant Virus

### (1) Obtainment of Gene and Introduction into Transfer Vector

Cloning of mGalT III (Mus musculus UDP-Gal: betaGlcNAc beta 1,4-galactosyltransferase, polypeptide 3) was performed by introducing amplified products obtained by PCR using cDNA (Accession No: NM_020579) as a template and the primer sets shown in Table 3 into the restriction enzyme sites shown in Table 4 in the transfer vector (pV01, manufactured by Sysmex Corporation).

ALP was used as a protein to which a desired complex-type sugar chain is added. Cloning of ALP gene was performed by introducing amplified products obtained by PCR using the synthesized nucleic acid represented by SEQ ID NO: 11 (GenScript) as a template and the primer sets shown in Table 3 into the restriction enzyme sites shown in Table 4 in the transfer vector (pM31, manufactured by Sysmex Corporation).

Cloning of hST (Homo sapiens ST6 beta-galactosamide alpha-2,6-sialyltranferase 1(ST6GAL1)) (Accession No: NM_173216) was performed by introducing gene sequences amplified by KOD-PCR (TOYOBO) using Human Universal QUICK-Clone (trademark) cDNA II (Clontech) as a template and the primer sets shown in Table 3 into the restriction enzyme sites shown in Table 4 in the transfer vector (pM31, manufactured by Sysmex Corporation).

### (2) Production of Recombinant Virus

BmN cells (Maeda et al, InverterbrateCell system and Applications, Vol. 1, p.167-181, CRC Press, Boca Raton (1989)) were co-transfected with any one of the four plasmids (0.5 µg) obtained in the above manner and DNA of linear baculovirus ABvNPV (0.2 µg) using a lipofection reagent (X-tremeGENE HP DNA transfection reagent: manufactured by Roche). Recombinant viruses were selected by limiting dilution using a 96-well plate and the culture supernatants were collected. The obtained BmFDL inhibitory antibody and mGalT III-, ALP-, and hST-expressing recombinant viruses were designated as BmFDL inhibitory antibody-NPV, GalT III-NPV, ALP-NPV, and ST-NPV, respectively.

### Example: Production of ALP Having Complex-Type Sugar Chain with Sialic Acid at Non-Reducing End

5th instar 1 st day silkworm larvae were infected with baculoviruses under the conditions shown in Table 6 below.

**[Table 6]**

| Samples | Baculovirus (NPV) infection conditions (PFU/ml) | | | |
|---|---|---|---|---|
| | ALP-NPV | GalT III-NPV | ST-NPV | BmFDL inhibitory antibody-NPV |
| A | 5 × 10⁷ | 1 × 10⁷ | 2 × 10⁷ | - |
| B | 5 × 10⁷ | 1 × 10⁷ | 2 × 10⁷ | 2 × 10⁶ |
| C | 5 × 10⁶ | 1 × 10⁷ | 2 × 10⁷ | - |
| D | 5 × 10⁶ | 1 × 10⁷ | 2 × 10⁷ | 2 × 10⁶ |

CMP sialic acid (manufactured by Yamasa Corporation) was transdermally administered to the resulting silkworm larvae (5th instar, 5th day) through an injection needle. The body fluid of the silkworm exhibiting infection symptoms was collected, and expressed ALPs were purified by Dock-Tag affinity purification.

### (Measurement of Amount of Sialic Acid Added)

Each of the ALPs (corresponding to 0.25 µg for each) obtained in the above manner was diluted with a buffer (25 mM Tris-HCl, pH 8.0, 10% glycerol, 1 mM CaCl₂, and 5 mM EGTA) to a final concentration of 2.5 µg/ml. The diluted solution was immobilized on a 96-well plate (Nunc immunoplate Maxisoap) at room temperature for 1 hour. The plate was washed 3 times with TTBS, so that blocking was performed at room temperature for 1 hour. Then, the plate was washed 3 times with TTBS. The lectin (SNA, manufactured by Vector Laboratories) labeled using a peroxidase-labeling kit (Dojindo Laboratories) in accordance with the protocol attached to the kit was applied at an amount of 100 µl to each well at a concentration of 2 µg/ml, followed by being allowed to stand at room temperature for 1 hour. Each resulting product was washed 3 times with TTBS, and then HRP was detected. The amounts of sialic acid added to samples A, B, C, and D were compared.

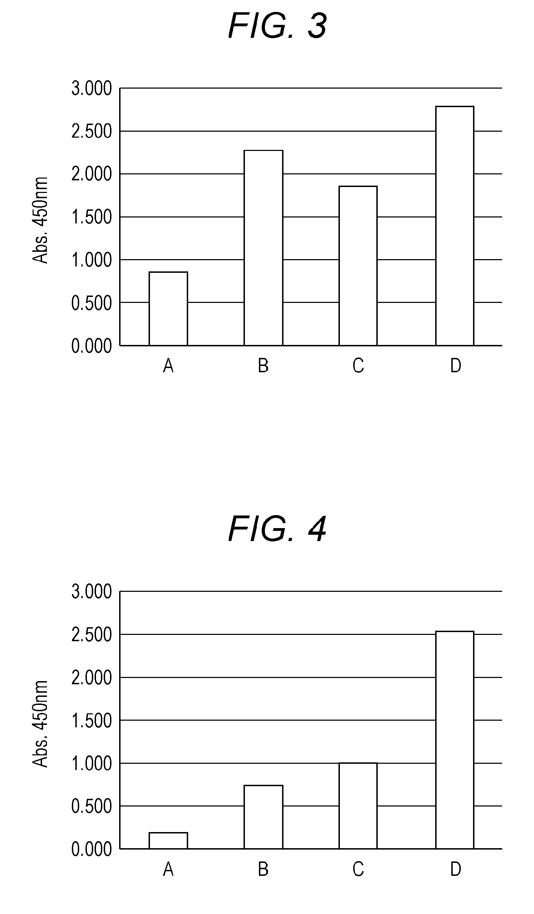

The results are shown in Fig. 3. From Fig. 3, it was found that the ratio of sialic acid to be added to the non-reducing end of the complex-type sugar chain is increased by twice or more based on an absorbance of 450 nm.

### (Measurement of Amount of Galactose Added)

Each of the ALPs formed in the above manner (corresponding to 0.25 µg for each) was diluted with a buffer (25 mM Tris-HCl, pH 8.0, 10% glycerol, 1 mM CaCl₂, and 5 mM EGTA) to a final concentration of 2.5 µg/ml. The diluted solution was immobilized on a 96-well plate (Nunc immunoplate Maxisoap) at room temperature for 30 minutes, and heated at 80°C for 2 hours for terminal sialic acid removal. The plate was washed 3 times with TTBS, so that blocking was performed at room temperature for 1 hour. Then, the plate was washed 3 times with TTBS. The lectin (SNA, manufactured by Vector Laboratories) labeled using a peroxidase-labeling kit (Dojindo Laboratories) in accordance with the protocol attached to the kit was applied at an amount of 100 µl to each well at a concentration of 2 µg/ml, followed by being allowed to stand at room temperature for 1 hour. Each resulting product was washed 3 times with TTBS, and then HRP was detected. The amounts of galactose added to samples A, B, C, and D were compared.

The results are shown in Fig. 4. Consequently, it was found that the ratio of galactose at the non-reducing end of the complex-type sugar chain after sialic acid removal is increased by twice or more based on an absorbance of 450 nm.

## Claims

1. A method of producing a glycoprotein, comprising the steps of:
introducing into an insect organism or insect cells a gene encoding a desired protein and a gene encoding an antibody, the antibody inhibiting a decomposing enzyme preventing formation of a desired complex-type sugar chain in the desired protein; and
obtaining a desired protein having a desired complex-type sugar chain from the insect organism or insect cells obtained in the introduction step.

2. The production method according to claim 1, wherein the decomposing enzyme is a membrane protein acting in cells.

3. The production method according to claim 1 or 2, wherein the decomposing enzyme is N-acetylglucosaminidase.

4. The production method according to any one of claims 1 to 3, wherein the desired complex-type sugar chain is a complex-type sugar chain with galactose or sialic acid linked to at least one non-reducing end.

5. The production method according to any one of claims 1 to 4, further comprising the step of introducing at least one selected from the group consisting of a gene encoding galactose transferase and a gene encoding sialyltransferase into the insect organism or insect cells.

6. The production method according to any one of claims 1 to 5, further comprising the step of introducing at least one selected from the group consisting of sialic acid and a sialic acid derivative into the insect organism or insect cells.

7. The production method according to any one of claims 1 to 6, further comprising the step of administering at least one selected from the group consisting of deoxygalactonojirimycin, methyl β-galactopyranoside and lactose into the insect organism or insect cells.

8. The production method according to any one of claims 1 to 7, wherein at least one selected from the group consisting of the gene encoding a desired protein and the gene encoding an antibody is inserted into at least one vector.

9. The production method according to any one of claims 1 to 8, wherein the insect is a lepidopteran insect.

10. The production method according to any one of claims 1 to 9, wherein the insect is Bombyx mori.

11. A vector comprising a gene encoding an antibody that inhibits activity of a membrane protein suppressing formation of a desired sugar chain in a desired protein.

12. A kit comprising:
a vector comprising a gene encoding an antibody that inhibits activity of a membrane protein suppressing formation of a desired sugar chain in a desired protein; and
a vector comprising a gene encoding a desired protein.

13. An insect organism or insect cell into which a vector comprising a gene encoding an antibody and a vector comprising a gene encoding a desired protein are introduced,
wherein the antibody inhibits activity of a membrane protein suppressing formation of a desired sugar chain in a desired protein.


SEQUENCE LISTING

<110>  SYSMEX CORPORATION

<120>  Method of producing glycoprotein, and vector, kit, insect organism
and insect cells

<130>  SYSM-107-EP

<150>  JP 2015-075070
<151>  2015-04-01

<160>  20

<170>  PatentIn version 3.5

<210>  1
<211>  33
<212>  DNA
<213>  Artificial Sequence

<220>
<223>  F primer for gamma chain of an inhibitory antibody

<400>  1
ccagatctta tgagcactga acacagacac ctc                                    33


<210>  2
<211>  34
<212>  DNA
<213>  Artificial Sequence

<220>
<223>  R primer for gamma chain of an inhibitory antibody

<400>  2
gggatatcct atttaccagg agagtgggag aggc                                   34


<210>  3
<211>  32
<212>  DNA
<213>  Artificial Sequence

<220>
<223>  F primer for kappa chain of an inhibitory antibody

<400>  3
ggcagatctt atggttttca cacctcagat ac                                     32


<210>  4
<211>  31
<212>  DNA
<213>  Artificial Sequence

<220>
<223>  R primer for kappa body of an inhibitory antibody

<400>  4
ccgatatcct aacactcatt cctgttgaag c                                      31

<210>  5
<211>  31
<212>  DNA
<213>  Artificial Sequence

<220>
<223>  F primer for ALP

<400>  5
caggtaccgg tacctaggta aaacaaaatg c                                    31


<210>  6
<211>  28
<212>  DNA
<213>  Artificial Sequence

<220>
<223>  R primer for ALP

<400>  6
ggtctagagt cagacaagcc tgcagggg                                        28


<210>  7
<211>  30
<212>  DNA
<213>  Artificial Sequence

<220>
<223>  F primer for mGalTIII

<400>  7
gaggatatca tgttgcggag gctgctggag                                      30


<210>  8
<211>  32
<212>  DNA
<213>  Artificial Sequence

<220>
<223>  R primer for mGalTIII

<400>  8
gactctagat tagtgtgagc cacggggagc tg                                   32


<210>  9
<211>  31
<212>  DNA
<213>  Artificial Sequence

<220>
<223>  F primer for hST

<400>  9
catcgagctc atgattcaca ccaacctgaa g                                    31


<210>  10
<211>  32

<212>  DNA
<213>  Artificial Sequence

<220>
<223>  R primer for hST

<400>  10
ctgtctagag cagtgaatgg tccggaagcc ag                                    32


<210>  11
<211>  1618
<212>  DNA
<213>  Artificial Sequence

<220>
<223>  A synthesized ALP

<400>  11
ggtacctagg taaaacaaaa tgcaatgggc ttgcgtgtta ctcttgcttg gcttgtggct      60

ccagctctca ctgacactga taccagcaga ggaagagaac cctgccttct ggaaccgtca     120

agcagcccaa gctctggacg tagctaagaa gctacagccc attcagacag ccgctaagaa     180

cgtgatctta ttccttggtg atggcatggg cgtcccaacg gttactgcca ctcgaatcct     240

caagggacag atgaacggca agttgggacc agaaacgcct ttagcgatgg accaattccc     300

ctatgttgcc ctgtccaaga cctacaacgt ggatcgccaa gtaccagaca gtgcgggtac     360

tgctacggcc tatctctgcg gtgtgaaagg gaactaccgt accattggag tgtcggctgc     420

agcccgttac aatcagtgca acacaaccag gggaaacgag gtcacctcag taatcaacag     480

ggcgaagaag gcggggaaag ctgttggagt ggtgacaact actcgcgtgc aacatgcttc     540

accagctggt gcttatgcac acacggtcaa ccgcaattgg tacagtgatg cggatctccc     600

agcagatgct cagaagaacg gatgccaaga catcgcggca cagctcgttt acaacatgga     660

catcgatgtg atactcggtg gaggtaggat gtacatgttc cctgagggaa cacctgatcc     720

tgagtaccct gacgatgctt ctgtcaatgg agtccgaaag gacaagcaaa atctggtaca     780

ggagtggcaa gccaaacacc aaggcgcgca gtacgtatgg aatcgcacag cccttctaca     840

ggctgccgac gactcttctg tcacccatct gatgggtctg tttgaacccg ctgacatgaa     900

atacaacgtc cagcaggatc acacgaagga tccgactctc gcagaaatga ccgaagctgc     960

cctacaggtc ctcagtagaa acccgcgtgg cttttacctg ttcgtcgaag gtggccgcat    1020

tgaccacggt caccatgacg gtaaagcgta catggcgctt accgaagcca tcatgttcga    1080

caatgccatc gccaaagcta acgagttgac tagcgaactg gacacactga tcttagtgac    1140

tgcagaccac tcccacgtgt tcagctttgg aggttatacc ctgaggggca cctccatttt    1200

cggccttgcc ccaggcaaag ccttagattc gaagagctac acctccattc tgtatgggaa    1260

tggccccgga tatgccttgg gaggaggcag tagacccgac gttaacggct ctactagcga    1320

ggagccaagc taccgccaac aggctgctgt tcccctggca tccgagacac atggtggtga     1380

ggatgtagca gtgttcgcta gaggtccgca agcgcacctg gtgcatggcg tccaggaaga     1440

aacctttgtt gcccacatca tggcattcgc tggttgtgtc gagccttaca cggactgtaa     1500

tctccccgcc cctgcaggct tgtctgacgc tgctcacttg gcggcctcag cgccctcgct     1560

agctttgttg gccggtgcaa tgctgctact gttggctccg gctctttact aatctaga       1618

<210>  12
<211>  24
<212>  DNA
<213>  Artificial Sequence

<220>
<223>  5'-RACE primer

<400>  12
gtaccaggcg tccacgtgcg agat                                              24

<210>  13
<211>  27
<212>  DNA
<213>  Artificial Sequence

<220>
<223>  3'-RACE primer

<400>  13
ccagagctca ctctgctttg ctcatcg                                           27

<210>  14
<211>  20
<212>  DNA
<213>  Artificial Sequence

<220>
<223>  F primer for sequencing

<400>  14
acgacgttgt aaaacgaggg                                                   20

<210>  15
<211>  20
<212>  DNA
<213>  Artificial Sequence

<220>
<223>  R primer for sequencing

<400>  15
tcacacagga aacagctatg                                                   20

<210>  16
<211>  31
<212>  DNA
<213>  Artificial Sequence

<220>
<223>  F primer for amplifying full length BmFDL

<400>  16
gttagatcta tgaagatgat gtcgtggggt g                                    31


<210>  17
<211>  26
<212>  DNA
<213>  Artificial Sequence

<220>
<223>  R primer for amplifying full length BmFDL

<400>  17
tctcgagact agagccacgc gtgcgg                                          26


<210>  18
<211>  31
<212>  DNA
<213>  Artificial Sequence

<220>
<223>  Primer 1 for cloning of gamma chain of 1-404-1

<400>  18
gagcggccgc atggcttggg tgtggacctt g                                    31


<210>  19
<211>  22
<212>  DNA
<213>  Artificial Sequence

<220>
<223>  Primer 2 for cloning of gamma chain of 1-404-1

<400>  19
tgaggagact gtgagagtgg tg                                              22


<210>  20
<211>  30
<212>  DNA
<213>  Artificial Sequence

<220>
<223>  Primer 1 for cloning of kappa chain of 1-404-1

<400>  20
ggagatctat ggagacagac acactcctgc                                      30